# EUROPEAN PATENT SPECIFICATION

(11) **EP 0 956 024 B1**
(45) Date of publication and mention of the grant of the patent: **21.07.2004**
(21) Application number: 97936149.0
(22) Date of filing: 23.07.1997
(51) Int. Cl.: A61K 31/57, A61K 31/565, A61P 15/18

(54) **MONOPHASIC ORAL CONTRACEPTIVE METHOD AND KIT COMPRISING A COMBINATION OF PROGESTIN AND ESTROGEN**
ORALE EINSTUFIGE EMPFÄNGNISVERHÜTUNGSMETHODE UND KOMBINATIONSPRÄPARAT DAS GESTAGEN UND ESTROGEN ENTHÄLT
METHODE DE CONTRACEPTION ORALE MONOPHASIQUE ET KIT COMPRENANT UNE ASSOCIATION DE PROGESTINE ET D'OESTROGENE

(30) Priority: 26.07.1996 US 686790
(43) Date of publication of application: 17.11.1999
(73) Proprietor: Wyeth, Madison, New Jersey 07940-0874 (US)
(72) Inventor: GAST, Michael, Jay, Phoenixville, PA 19460 (US)
(74) Representative: Mannion, Sally Kim, Dr.
(86) International application number: PCT/US1997/012795
(87) International publication number: WO 1998/004269

(56) References cited:
- EP-A- 0 491 415
- WO-A-95/17194
- WO-A-97/01342
- DE-A- 4 411 585
- OELKERS W ET AL: "Effects of a new oral contraceptive containing an antimineralocorticoid progestogen, drospirenone, on the renin-aldosterone system, body weight, blood pressure, glucose tolerance, and lipid metabolism." J CLIN ENDOCRINOL METAB, JUN 1995, 80 (6) P1816-21, UNITED STATES, XP002046619
- OELKERS W K H: "Effects of estrogens and progestogens on the renin-aldosterone system and blood pressure" STEROIDS, 61 (4). 1996. 166-171., XP002046622
- OETTEL M. ET AL: "A 19-norprogestin without 17alpha-ethinyl group II: Dienogest from a pharmacodynamic point of view" DRUGS OF TODAY, 1995, 31/7 (517-536), SPAIN, XP002046623
- KUHL H.: "Current developments in hormonal contraception" GYNAKOLOGE, 1992, 25/4 (231-240), GERMANY, XP002046624

## Description

### BACKGROUND OF THE INVENTION

The vast majority of oral contraceptives consist of a combination of a progestin and estrogen that are administered concurrently for 21 days followed either by a 7 day pill free interval or by the administration of a placebo for 7 days in each 28 day cycle. The most important aspects of a successful oral contraceptive product are effective contraception, good cycle control (absence of spotting and breakthrough bleeding and occurrence of withdrawal bleeding), and minimal side effects. Combination oral contraceptives have traditionally acted by suppression of gonadotropins. In addition, it appears that the progestin component is primarily responsible for contraceptive efficacy through inhibition of ovulation, and other peripheral effects which include changes in the cervical mucus (which increase the difficulty of sperm entry into the uterus) and the endometrium (which reduce the likelihood of implantation). The estrogenic component intensifies the anovulatory effect of the progestin, and is also important for maintaining cycle control.

Since the introduction of oral contraceptives (OCs) over a quarter-century ago, research has been directed toward developing preparations that minimize the potential for side effects while maintaining efficacy and normal menstrual patterns. The first-generation OCs contained more progestin and estrogen than was necessary to prevent conception. Adverse hemostatic and metabolic changes, clinical problems, and side effects were associated with these high-dose preparations. In 1978, the World Health Organization (WHO) recommended that the focus of OC research should be the development of products containing the lowest possible dose levels of estrogen and progestin.

The first reductions in steroid content in a combination pill were focused on estrogen because it, rather than progestin, was thought to be related to the most serious side effects. Reduction in progestin content followed, as evidence mounted that lowering progestin intake might lower the risk of cardiovascular complications such as stroke and ischemic heart disease. [Kay CR, Am J Obstet Gynecol 142:762 (1982)). However, this evidence was not as clear as that implicating estrogen in thromboembolic disorders. [Inman WHW, Br Med J 2:203 (1970); Stolley PD, Am J Epidemiol 102:197 (1975)]. The need for a balance between estrogens and progestins to minimize adverse effects on carbohydrate metabolism and on lipid and lipoprotein levels was also recognized. [Bradley DD, N Engl J Med 299:17 (1978); Wynn V, Lancet 1:1045 (1979)]. Researchers then found that the synergistic action between progestin and estrogen in a balanced ratio successfully inhibited ovulation at low levels of both components.

Research into low-dose progestins was advanced significantly by the development of norgestrel (Ng) and levonorgestrel (LNg). Levonorgestrel is the biologically active moiety of racemic norgestrel. It is strongly progestational, has no inherent estrogenic activity, is antiestrogenic, and possesses good biologic activity. The contraceptive effects of levonorgestrel are manifested throughout the hypothalamic-pituitary-gonadal-target organ axis.

Ethinyl estradiol (EE) is the estrogen most frequently used in combination OCs. In attempts to fulfill the WHO objective, the dosage of EE in marketed OC formulations has been steadily reduced from that found in earlier OCs. Thromboembolic mortality decreased when the amount of synthetic estrogen in OC formulations was reduced from 100 µg to 50 µg. Subsequently, a significant reduction in fatal myocardial infarctions was reported for women using OCs with 30 µg of EE rather than 50 µg of EE. [Meade TW, Br Med J 280:1157 (1980)].

In keeping with the goal of reducing the total steroidal dosage, while maintaining contraceptive efficacy, good cycle control, and minimizing side effects, numerous regimens have been developed in which the progestin/estrogen combination is administered either as a fixed dosage combination (monophasic) or as biphasic or triphasic regimens in which the dosage of the combination is varied either once or twice throughout the menstrual cycle. In these regimens, the progestin/estrogen combination is typically administered for 21 days followed by either a 7-day pill free period or the administration of a non-contraceptive placebo (or iron supplement) for 7 days. In these regimens, 3-ketodesogestrel (3-KDSG), desogestrel (DSG), levonorgestrel (LNg), gestodene (GTD), norgestrel (NG), and norethindrone (NE) are typically used as the progestin while ethinyl estradiol (EE); 17β-estradiol, and mestranol are typically the estrogenic components. Other progestins less frequently used include drospirenone (DRSP) and dienogest (DGST).

An oral contraceptive product containing a combination of 3 mg DGST and 30 µg EE for 21-day administration per cycle is marketed in Germany.

Several examples of attempts at reducing the total steroidal dosage are provided below.

Spona (PCT Publication WO 95/17194) discloses contraceptive regimens which consist of the administration of a combinaton of a progestin (50 - 75 µg GTD, 75 - 125 µg LNg, 60 - 150 µg DSG, 60 - 150 µg 3-KDSG. 100 - 300 µg DRSP, 100 - 200 µg cyproterone acetate, 200 - 300 µg norgestimate, or >350 - 750 µg norethisterone) and an estrogen (15 - 20 µg EE or 2 - 6 mg 17β-estradiol) for 23-24 days per cycle.

Oettel (EP 628,312 A1) discloses combination contraceptive combinations containing the combination of three components: a biogenic estrogen (estradiol, estrone, or estriol), a synthetic estrogen (EE or mestranol), and a progestin (LNg, desogestrel, progesterone, norethisterone acetate, DGST, chlormadinone acetate, gestodene, or cyproterone acetate). In one embodiment, the combination is administered for 21 days followed by the administration of placebo (or pill free) or an estrogen on days 22-28 of the cycle.

Oettel (EP 696,454 A2) discloses a three phase contraceptive regimen in which the first phase consists of the administration for 3-4 days of a composition containing at least one biogenic estrogen; the second phase consists of the administration for 20-22 days of at least one biogenic estrogen and at least one progestin (progesterone, DGST, desogestrel, 3-KDSG, GTD, LNg, norgestimate, notethisterone, norethisterone acetate, dehydrogestrone, chloromadinone acetate, cyproterone acetate, medroxyprogesterone acetate, or megestrol acetate); and the third phase consists of the administration for 3-4 days of a composition containing at least biogenic one estrogen.

Lachnit (PCT Publication WO 95/26730) discloses bridged regimens consisting of the administration of a combination of a progestin/estrogen combination (50 - 125 µg LNg and 10 - 40 µg EE) for the first 23-24 days of the menstrual cycle followed by the administration of an estrogen (2 - 40 µg EE) for 4-10 days for a total administration of at least 28 days per cycle. The use of 100 - 300 µg drospirenone and 10 - 40 µg EE as the 23-24 day progestin/estrogen combination is disclosed. Lachnit also discloses a triphasic plus bridging regimen (4-9 days, 4-9 days, 9-13 days, and 28 days for the three phases and estrogen phase, respectively) in which a combination of 50 µg LNg and 20 µg EE are administered in the first phase, a combination of 75 µg LNg and 25 µg EE are administered in the second phase, a combination of 100 µg LNg and 20 µg EE are administered in the third phase, and 10 µg EE is administered in the estrogen phase. Other progestins disclosed include GTD, DSG, 3-KDSG, DRSP, cyproterone acetate, norgestimate, and norethisterone.

Upton (EP Patent Specification 253,607 B1) teaches the use of low dose progestin/estrogen combinations for combined hormone replacement therapy and contraception in climacteric women. Climacteric women are defined in Upton as pre-menopausal women around 40 years of age whose hormone levels are waning. The climacteric woman still ovulates (albeit may have irregular ovulation), but she still experiences many of the symptoms of the hypoestrogenic menopausal woman, such as insomnia, hot flushes, and irritability. Upton teaches the administration of a 23-26 day monophasic regimen of progestin/estrogen followed by a pill free or placebo interval of 2-5 days; with 24 days of progestin/estrogen administration followed by a 4-day pill free or placebo administration being preferred. Upton teaches the use of a progestin selected from 25 - 100 µg LNg, 10 - 70 µg GTD, 25 - 100 µg DSG, 25 - 100 µg 3-KDSG, and 85 - 350 µg NE used in combination with an estrogen selected from 500 - 2000 µg 17β-estradiol, 8 - 30 µg EE, and 15 - 60 µg mestranol. Based on relative potencies, Upton teaches that a dose of 75 µg LNg is equivalent to 35 µg of GTD, 75 µg of 3-KDSG or DSG, and 250 µg NE and that a dose of 1000 µg of 17β-estradiol is equivalent to a dose of 15 µg EE and 30 µg mestranol. Upton also teaches that NG may be substituted for LNg, but at twice the dose.

Sartoretto (Clinica e Terapeutica 3: 399 (1974)) discloses a monophasic contraceptive regimen consisting of the administration of a combination 100 µg LNg and 20 µg EE for 21 days.

Lachnit-Fixson (U.S. Patent 3,969,502) discloses biphasic progestin/estrogen combination regimens in which a combination of 50-125 µg LNg and 25-35 µg EE are administered for 10-12 days in the first phase and 100-350 µg LNg and 30-50 µg EE are administered for 10-12 days in the second phase. Placebo is administered for 5-7 days following the administration of the contraceptive steroid regimen.

Lachnit-Fixson (U.S. Patent 3,957,982) discloses triphasic 21-day progestin/estrogen regimens in which a combination of 40-90 µg LNg and 20-50 µg EE is administered for 4-6 days in the first phase, 50-125 µg LNg and 30-50 µg EE is administered for 4-6 days in the second phase, and 100-250 µg LNg and 25-50 µg EE is administered for 9-11 days in the third phase. It is preferred that the first, second, and third phases are 6, 5, and 10 days, respectively.

Bennick (U.S. Patent 5,418,228) discloses triphasic regimens which consist of the administration of a combination progestin/estrogen in a 6-8 day phase, a second 6-8 day phase, and a third 6-8 day phase, with it being preferred that the three contraceptive steroid phases be 7 days each. Bennick discloses that the first contraceptive steroid phase consists of a progestin at a daily dosage equivalent to 75 - 150 µg DSG and an estrogen at a daily dosage equivalent to 20 - 25 µg EE; the second contraceptive steroid phase consists of a progestin at a daily dosage equivalent to 75 - 125 µg DSG and an estrogen at a daily dosage equivalent to 20 µg EE; and the third contraceptive steroid phase consists of a progestin at a daily dosage equivalent to 75 - 100 µg DSG and an estrogen at a daily dosage equivalent to 20 µg EE. Placebo is administered for 7 days following the 21-day contraceptive steroid period. Bennick discloses that the progestin may be 3-KDSG, DSG, LNg, or GTD.

Bergink (U.S. Patent 5,262,408) discloses a 24 day triphasic combination regimen in which the first 7-9 day phase consists of the administration of a progestin at a daily dosage equivalent to 100 µg DSG and an estrogen at a daily dosage equivalent to 25 µg EE, the second 7-9 day phase consists of the administration of a progestin at a daily dosage equivalent to 125 µg DSG and an estrogen at a daily dosage equivalent to 20 µg EE, and the third 7-9 day phase consists of the administration of a progestin at a daily dosage equivalent to 50 µg DSG and an estrogen at a daily dosage equivalent to 20 µg EE. It is preferred that the three phases be 8 days each. Following the 24 day contraceptive steroid administration, a placebo may be administered for 4 days, the 4 day interval may be pill free, or a progestin at a dosage equivalent to 25-35 µg DSG may be administered.

Boissonneault (U.S. Patent 4,962,098) discloses triphasic progestin/estrogen combinations in which the amount of the estrogenic component is increased stepwise over the three phases. Contraceptive steroid combinations are taken for 4-7 days during the first phase (5 days being preferred); for 5-8 days during the second phase (7 days preferred); and for 7-12 days during the third phase (9 days being preferred). Following the administration of 21-days of the contraceptive steroid combination, placebo is taken for 7 days. For all three phases, 0.5-1.5 mg of norethindrone acetate is used in the progestin, with 1 mg being preferred. 10-30 µg EE is used in the first phase, 20-40 µg in the second, and 30-50 µg in the third phase.

Pasquale (U.S. Patent 4,921,843) discloses combination progestin/estrogen contraceptive regimens which contain 0.5 to I mg of progestin and an estrogen having a dose equivalent to 10-40 µg of EE. NE, LNg, D-17β-acetoxy-13β-ethyl-17α-ethinyl-gon-4-en-3-one oxime, and 19-nor-17-hydroxy progesterone ester are disclosed as progestins, with NE being preferred- Specifically disclosed regimens include a uniphasic regimen (2 days of placebo, 5 days of 20 µg EE, and 21 days of a combination of 500 µg NE and 35 µg EE); a uniphasic regimen (2 days of placebo, 5 days of 40 µg EE, and 21 days of a combination of 500 µg NE and 35 µg EE); and a triphasic regimen (2 days of placebo; 5 days of 20-40 µg EE; 7 days of a combination of 500 µg NE and 35 µg EE; 7 days of a combination of 750 µg NE and 35 µg EE; and 7 days of a combination of 1 mg NE and 35 µg EE).

Pasquale (U.S. Patent 4,628,051) discloses triphasic progestin/estrogen combination regimens in which contraceptive steroid is administered for 21 days. Contraceptive steroid combinations are taken for 5-8 days during the first phase (7 days being preferred); for 7-11 days during the second phase (7 days preferred); and for 3-7 days during the third phase (7 days being preferred). In all three phases, an estrogen at a daily dosage equivalent to 20-50 µg EE is administered in combination with a progestin having a daily dosage equivalent to 65-750 µg NE in the first phase, 0.25-1.0 mg NE in the second phase, and 0.35-2.0 mg NE in the third phase. A specific triphasic regimen discloses the administration of 35 µg EE in each of the three 7-day phases in combination with 0.5 mg, 0.75 mg, and 1.0 mg in the first, second, and third phases, respectively. A second specific triphasic regimen discloses the administration of 35 µg EE in each of the three 7-day phases in combination with 50 µg, 75 µg, and 100 µg in the first, second, and third phases, respectively. A third specific triphasic regimen discloses the administration of 35 µg EE in each of the three 7-day phases in combination with 25 µg, 35, µg, and 50 µg in the first, second, and third phases, respectively.

Lachnit-Fixson (U.S. Patent 4,621,079) discloses triphasic 21-day progestin/estrogen combination regimens in which a combination of 40-70 µg GTD and 20-35 µg EE is administered for 4-6 days in the first phase; 50-100 µg GTD and 30-50 µg EE is administered for 4-6 days in the second phase; and 80-120 µg GTD and 20-50 µg EE is administered for 9-11 days in the third phase. Placebo is administered for 7 days following the 21-day contraceptive steroid regimen.

Pasquale (U.S. Patent 4,530,839) discloses triphasic 21-day progestin/estrogen combination regimens in which a dose of 20-50 µg EE is administered in all three phases in combination with a contraceptively effective daily dose of progestin in the first phase, 1.5-2 times that dose of progestin in the second phase, and 2-2.5 times the first phase dose of progestin in the third phase. Each of the three phases is 7 days long. A specific regimen discloses 20-50 µg EE in combination with 500 µg LNg, 750 µg LNg, and 1 mg LNg during each of the three 7-day phases, respectively.

Edgren (U.S. Patent 4,390,531) discloses triphasic 21-day progestin/estrogen combination regimens in which a dose of 20-40 µg EE (or another estrogen in an equivalent dosage) is administered in all three phases in combination with 0.3-0.8 mg NE (or another progestin in an equivalent dosage) for 5-8 days in the first phase, twice the dose of NE for 7-11 days in the second phase, and the dose of NE being the same as in the first phase for 3-7 days in the third phase. It is preferred that each of the three phases is 7 days. Placebo is administered for 6-8 days following administration of the contraceptive steroid combination. A specific regimen discloses a first phase of 7 days of 0.5 mg NE in combination with 35 µg EE, a second 7 day phase of 1.0 mg NE in combination with 35 µg EE, and a third 7 day phase of 0.5 mg NE in combination with 35 µg EE.

Moore (DE 4313926 A1) discloses bridged triphasic regimens consisting of the administration of a combination of 10 - 50 µg LNg and 5 - 20 µg EE from days 1-7 of the menstrual cycle; of 50 - 75 µg LNg and 5 - 20 µg EE from days 8-14 of the menstrual cycle; of 75 - 125 µg LNg and 5 - 20 µg EE from days 15-21 of the menstrual cycle; and 5 - 20 µg EE from days 22-28 of the menstrual cycle.

Erlich (German Patent DE 4,104,385 C1 and U.S. Patent 5,280,023) discloses sequential contraceptive regimens consisting of the administration of an estrogen which effects a disturbance of follicle stimulation, followed by the administration of a combination of a progestin/estrogen in a dose at least adequate to inhibit ovulation. The regimen is administered for a total of 28 days per cycle. It is preferred that the estrogen is administered for 5-14 days per cycle and the progestin/estrogen combination is administered for 23-14 days per cycle, so that the total administration is for 28 days per cycle. Specific regimens include (a) 4 mg estradiol for 7 days followed by 21 days of the combination of 1 mg norethisterone acetate and 4 mg estradiol; (b) 2 mg estradiol valerate for 7 days followed by 21 days of the combination of 2 mg chlormadinone acetate and 4 mg estradiol valerate; and (c) 20 µg EE followed by 18 days of the combination of 150 µg LNg and 20 µg EE. Regimen (c) in Erlich provides a total steroidal load of 2.7 mg of LNg and 560 µg EE per 28 day cycle.

WO 97/01342 discloses a two-stage combined preparation for hormonal contraception containing at least 30 daily dosages, the first stage being 25 to 77 doses of combined estrogen and progestin and the second stage being 5 to 7 doses of estrogen only.

Oettel et al (Drugs of Today, 1995, 31(7), 517-536) describes the properties of dienogest as a progestogen and its use in combination with EE as a contraceptive.

### DESCRIPTION OF THE INVENTION

This invention provides a monophasic combination progestin/estrogen oral contraceptive regimen for females of child-bearing age that provides effective contraception, good cycle control, and minimal side effects while greatly reducing the total contraceptive steroid administered (particularly the estrogenic component) per 28-day cycle. To achieve the substantial reduction in the total contraceptive steroid administered per cycle while maintaining good cycle control, the low dose progestin/estrogen combination is administered for 23-25 days per cycle. Administration of the contraceptive progestin/estrogen combination is begun on the first day of menses (day 1), and continued for 23-25 consecutive days. Following the 23-25-day administration period, a non-contraceptive placebo (devoid of progestins and estrogens) can be provided for 3-5 days to aid in compliance with the desired contraceptive regimen. Alternatively, the 3-5-day interval can be pill free. The total cycle length is 28 days.

In particular, this invention provides a method of contraception which comprises oral administration to a female of child bearing age a combination of a progestin at a daily dosage selected from 40-500 µg trimegestone and 250 µg -4 mg dienogest, and an estrogen at a daily dosage equivalent in estrogenic activity to 10-20 µg ethinyl estradiol for 23-25 days of a 28 day menstrual cycle beginning on day 1 of the menstrual cycle, wherein the same dosage of the progestin and estrogen combination is administered in each of the 23-25 days; followed by a non-contraceptive placebo for 3-5 consecutive days beginning on the day immediately following the last day of administration of the progestin/estrogen combination or a 3-5 day pill free interval, such that the total days of administration of the progestin/estrogen combination plus the non-contraceptive placebo or pill free interval equals 28 days

Preferred estrogens include ethinyl estradiol; 17β-estradiol; conjugated estrogens, USP; estrone or a salt thereof; and mestranol; with ethinyl estradiol being more preferred. When ethinyl estradiol is used as the estrogen, it is preferred that the daily dosage of ethinyl estradiol is 10-20 µg, with 15 µg being more preferred. Preferred salts of estrone include sodium and piperate salt. When conjugated estrogens, USP are used as the estrogen, it is preferred that the daily dosage is 0.3-5 mg, with a daily dose of 1.25 mg conjugated estrogens, USP being equivalent to a daily dose of 15 µg ethinyl estradiol.

Following the 23-25-day period, a non-contraceptive placebo, which may contain an iron supplement, such as 75 mg of ferrous fumerate, may be administered for 4 days (through day 28 of the menstrual cycle) or the 4 days following administration of the contraceptive combination may be pill free.

It is preferred that the progestin/estrogen combination be administered for 24 days beginning on day 1 of the menstrual cycle.

The following daily dosages of a combination of trimegestone and ethinyl estradiol are preferred for contraception when administered for 23-25 consecutive days beginning on the first day of menses. Regimen B is most preferred.

| PREFERRED DAILY DOSAGES | | |
|---|---|---|
| Regimen | Trimegestone | Ethinyl Estradiol |
| A | 250 µg | 15 µg |
| B | 125 µg | 15 µg |
| C | 75 µg | 15 µg |
| D | 50 µg | 15 µg |
| E | 40 µg | 15 µg |

The following daily dosages of a combination of dienogest and ethinyl estradiol are preferred for contraception when administered for 23-25 consecutive days beginning on the first day of menses. Regimen B is most preferred.

| PREFERRED DAILY DOSAGES | | |
|---|---|---|
| Regimen | Dienogest | Ethinyl Estradiol |
| A | 2 mg | 15 µg |
| B | 1 mg | 15 µg |
| C | 800 µg | 15 µg |
| D | 750 µg | 15 µg |
| E | 500 µg | 15 µg |

It is preferred that the combination progestin/estrogen contraceptive be administered in unit dosage form i.e., tablet or pill, with each unit providing the entire daily dosage. It is preferred that the progestin and estrogen are admixed together in the same dosage unit. Such dosage units can be prepared by conventional methodology that is well known to one skilled in the art. In each dosage unit, the contraceptively active progestin and estrogen are combined with excipients, vehicles, pharmaceutically acceptable carriers, and colorants. For example, the following illustrates an acceptable composition of a contraceptive progestin/estrogen combination of this invention.

### EXAMPLE 1

Trimegestone, 125 µg
Ethinyl estradiol, 15 µg
Microcrystaline Cellulose
Lactose, NF, Spray Dried
Polacrillin Potassium, NF
Magnesium Stearate
Opadry Pink
Polyethylene Glycol, 1500, Flakes
Water, Purified, USP
Wax E (Pharma)

This invention also provides a contraceptive kit adapted for daily oral administration which consists of 23-25 separate dosage units, said dosage units each consisting of a combination of 40-500 µg trimegestone or 250 µg - 4 mg dienogest, and an estrogen at a daily dosage equivalent in estrogenic activity to 10-20 µg ethinyl estradiol. Preferably, the contraceptive kit consists of 24 dosage units. In another embodiment, the contraceptive kit contains 28 daily dosage units with 3-5 being a non-contraceptive placebo, that may contain an iron supplement, such as 75 mg ferrous fumerate. The daily dosage arrangements are preferably arranged in a blister pack or in a dial pack type tablet dispenser.

## Claims

1. A method of contraception which comprises oral administration to a female of child bearing age a combination of a progestin at a daily dosage selected from 40-500 µg trimegestone and 250 µg -4 mg dienogest, and an estrogen at a daily dosage equivalent in estrogenic activity to 10-20 µg ethinyl estradiol for 23-25 days of a 28 day menstrual cycle beginning on day 1 of the menstrual cycle, wherein the same dosage of the progestin and estrogen combination is administered in each of the 23-25 days; followed by a non-contraceptive placebo for 3-5 consecutive days beginning on the day immediately following the last day of administration of the progestin/estrogen combination or a 3-5 day pill free interval, such that the total days of administration of the progestin/estrogen combination plus the non-contraceptive placebo or pill free interval equals 28 days

2. The method according to claim 1, wherein the estrogen is selected from ethinyl estradiol; 17β-estradiol; conjugated estrogens, USP; estrone or a salt thereof; and mestranol.

3. The method according to claim 2, wherein the estrogen is ethinyl estradiol.

4. The method according to claim 3, wherein the progestin/estrogen combination is administered for 24 days per menstrual cycle beginning on day 1 of the menstrual cycle.

5. The method according to claim 4 wherein the daily dosage of ethinyl estradiol is 15 µg.

6. The method according to claim 5, wherein the progestin is trimegestone.

7. The method according to claim 6 wherein the daily dosage oftrimegestone is 250 µg.

8. The method according to claim 6 wherein the daily dosage oftrimegestone is 125 µg.

9. The method according to claim 6 wherein the daily dosage oftrimegestone is 75 µg.

10. The method according to claim 6 wherein the daily dosage oftrimegestone is 50 µg.

11. The method according to claim 6 wherein the daily dosage oftrimegestone is 40 µg.

12. The method according to claim 5 wherein the progestin is dienogest.

13. The method according to claim 12 wherein the daily dosage of dienogest is 2 mg.

14. The method according to claim 12 wherein the daily dosage of dienogest is 1 mg.

15. The method according to claim 12 wherein the daily dosage of dienogest is 800 µg.

16. The method according to claim 12 wherein the daily dosage of dienogest is 750 µg.

17. The method according to claim 12 wherein the daily dosage of dienogest is 500 µg.

18. The method according to claim 1, in which a non-contraceptive placebo is administered for 3-5 consecutive days beginning on the day immediately following the last day of administration of the progestin/estrogen combination.

19. The method according to claim 18 in which the non-contraceptive placebo contains an iron supplement.

20. A contraceptive kit adapted for oral administration in a 28 day menstrual cycle beginning on day I of the menstrual cycle containing dosage units which consist of 23-25 separate dosage units, said dosage units each containing a combination of a progestin at a dosage selected from 40-500 µg trimegestone and 250 µg -4 mg dienogest, and an estrogen at a dosage equivalent in estrogenic activity to 10-20 µg ethinyl estradiol, wherein each of the dosage units contains the same dosage of progestin and estrogen, and optionally 3-5 dosage units each containing a non-contraceptive placebo.

21. The contraceptive kit according to claim 20 wherein the estrogen is selected from ethinyl estradiol; 17β-estradiol; conjugated estrogens, USP; estrone or a salt thereof; and mestranol.

22. The contraceptive kit according to claim 21 wherein the estrogen is ethinyl estradiol.

23. The contraceptive kit according to claim 20 in which the number of dosage units is 24.

24. A contraceptive kit adapted for oral administration according to claim 20 containing dosage units consisting of 28 separate dosage units, 23-25 of said dosage units each containing a combination of a progestin at a dosage selected from 40-500 µg trimegestone and 250 µg -4 mg dienogest, and an estrogen at a dosage equivalent in estrogenic activity to 10-20 µg ethinyl estradiol, wherein each of the 23-25 dosage units contains the same dosage of progestin and estrogen; and 3-5 of said dosage units each containing a non-contraceptive placebo.

## Patentansprüche

1. Verfahren der Empfängnisverhütung, welches umfasst: orale Verabreichung einer Kombination aus einem Progestin bei einer täglichen Dosierung, ausgewählt aus 40-500 µg Trimegeston und 250 µg -4 mg Dienogest, und einem Estrogen bei einer täglichen Dosierung, welche in estrogener Wirksamkeit äquivalent zu 10-20 µg Ethinylestradiol ist, an eine Frau im gebährfähigen Alter für 23-25 Tage eines 28-tägigen Menstruationszyklus, beginnend am Tag 1 des Menstruationszyklus, wobei die gleiche Dosierung der Progestin und Estrogen Kombination an jedem der 23-25 Tage verabreicht wird; gefolgt von einem nicht-empfängnisverhütenden Placebo für 3-5 aufeinander folgende Tage, beginnend am Tag direkt nach dem letzten Tag der Verabreichung der Progestin/Estrogen-Kombination oder einem 3-5 tägigen Pillen-freien Intervall, so dass die gesamten Tage der Verabreichung der Progestin/Estrogen-Kombination plus des nicht-empfängnisverhütenden Placebo oder Pillen-freien Intervalls 28 Tagen entsprechen.

2. Verfahren gemäß Anspruch 1, wobei das Estrogen ausgewählt wird aus Ethinylestradiol, 17β-Estradiol, konjugierten Estrogenen, USP; Estron oder einem Salz davon; und Mestranol.

3. Verfahren gemäß Anspruch 2, wobei das Estrogen Ethinylestradiol ist.

4. Verfahren gemäß Anspruch 3, wobei die Progestin/Estrogen-Kombination für 24 Tage pro Menstruationszyklus verabreicht wird, beginnend an Tag 1 des Menstruationszyklus.

5. Verfahren gemäß Anspruch 4, wobei die tägliche Dosierung von Ethinylestradiol 15 µg beträgt.

6. Verfahren gemäß Anspruch 5, wobei das Progestin Trimegeston ist.

7. Verfahren gemäß Anspruch 6, wobei die tägliche Dosierung von Trimegeston 250 µg beträgt.

8. Verfahren gemäß Anspruch 6, wobei die tägliche Dosierung von Trimegeston 125 µg beträgt.

9. Verfahren gemäß Anspruch 6, wobei die tägliche Dosierung von Trimegeston 75 µg beträgt.

10. Verfahren gemäß Anspruch 6, wobei die tägliche Dosierung von Trimegeston 50 µg beträgt.

11. Verfahren gemäß Anspruch 6, wobei die tägliche Dosierung von Trimegeston 40 µg beträgt.

12. Verfahren gemäß Anspruch 5, wobei das Progestin Dienogest ist.

13. Verfahren gemäß Anspruch 12, wobei die tägliche Dosierung von Dienogest 2 mg beträgt.

14. Verfahren gemäß Anspruch 12, wobei die tägliche Dosierung von Dienogest 1 mg beträgt.

15. Verfahren gemäß Anspruch 12, wobei die tägliche Dosierung von Dienogest 800 µg beträgt.

16. Verfahren gemäß Anspruch 12, wobei die tägliche Dosierung von Dienogest 750 µg beträgt.

17. Verfahren gemäß Anspruch 12, wobei die tägliche Dosierung von Dienogest 500 µg beträgt.

18. Verfahren gemäß Anspruch 1, wobei ein nicht-empfängnisverhütendes Placebo für 3-5 aufeinander folgende Tage verabreicht wird, beginnend am Tag direkt nach dem letzten Tag der Verabreichung der Progestin/Estrogen-Kombination.

19. Verfahren gemäß Anspruch 18, wobei das nicht-empfängnisverhütende Placebo einen Eisen-Zusatz enthält.

20. Empfängnisverhütungs-Kit, angepasst für orale Verabreichung in einem 28-tägigen Menstruationszyklus, beginnend an Tag 1 des Menstruationszyklus, welches Dosierungseinheiten enthält, welche aus 23-25 getrennten Dosierungseinheiten bestehen, wobei besagte Dosierungseinheiten jeweils eine Kombination aus einem Progestin bei einer Dosierung, ausgewählt aus 40-500 µg Trimegeston und 250 µg -4 mg Dienogest, und einem Estrogen bei einer Dosierung, welche in estrogener Wirksamkeit äquivalent zu 10-20 µg Ethinylestradiol ist, enthalten, wobei jede der Dosierungseinheiten die gleiche Dosierung an Progestin und Estrogen enthält und gegebenenfalls 3-5 Dosierungseinheiten jeweils ein nicht-empfängnisverhütendes Placebo enthalten.

21. Empfängnisverhütungs-Kit gemäß Anspruch 20, wobei das Estrogen ausgewählt wird aus Ethinylestradiol; 17β-Estradiol; konjugierten Estrogenen, USP; Estron oder einem Salz davon; und Mestranol.

22. Empfängnisverhütungs-Kit gemäß Anspruch 21, wobei das Estrogen Ethinylestradiol ist.

23. Empfängnisverhütungs-Kit gemäß Anspruch 20, in welchem die Anzahl an Dosierungseinheiten 24 beträgt.

24. Empfängnisverhütungs-Kit, angepasst für orale Verabreichung gemäß Anspruch 20, welches Dosierungseinheiten enthält, bestehend aus 28 getrennten Dosierungseinheiten, wobei 23-25 der besagten Dosierungseinheiten jeweils eine Kombination aus einem Progestin bei einer Dosierung, ausgewählt aus 40-500 µg Trimegeston und 250 µg -4 mg Dienogest, und einem Estrogen bei einer Dosierung, welche in estrogener Wirksamkeit äquivalent zu 10-20 µg Ethinylestradiol ist, enthalten, wobei jede der 23-25 Dosierungseinheiten die gleiche Dosierung an Progestin und Estrogen enthält und 3-5 der besagten Dosierungseinheiten jeweils ein nicht-empfängnisverhütendes Placebo enthalten.

## Revendications

1. Procédé de contraception qui comprend l'administration orale à une femme en âge de procréer d'une combinaison d'un progestatif à une dose quotidienne sélectionnée parmi 40-500 µg de trimégestone et 250 µg-4 mg de diénogest, et d'un oestrogène à une dose quotidienne équivalente au niveau de l'activité d'oestrogènes à 10-20 µg d'éthinyloestradiol pendant 23-25 jours d'un cycle menstruel de 28 jours en commençant au jour 1 du cycle menstruel, dans lequel la même dose de la combinaison de progestatif et d'oestrogène est administrée chacun des 23-25 jours; suivie par un placebo non contraceptif pendant 3-5 jours consécutifs en commençant le jour suivant immédiatement le dernier jour d'administration de la combinaison de progestatif/oestrogène ou par un intervalle de 3-5 jours sans pilule, de sorte que le total des jours d'administration de la combinaison de progestatif/oestrogène plus le placebo non contraceptif ou l'intervalle sans pilule vaut 28 jours.

2. Procédé suivant la revendication 1, dans lequel l'oestrogène est sélectionné parmi de l'éthinyloestradiol; du 17ß-oestradiol; des oestrogènes conjugués, USP; de l'oestrone ou un sel de celle-ci; et du mestranol.

3. Procédé suivant la revendication 2, dans lequel l'oestrogène est de l'éthinyloestradiol.

4. Procédé suivant la revendication 3, dans lequel la combinaison de progestatif/oestrogène est administrée pendant 24 jours par cycle menstruel en commençant au jour 1 du cycle menstruel.

5. Procédé suivant la revendication 4, dans lequel la dose quotidienne d'éthinyloestradiol est de 15 µg.

6. Procédé suivant la revendication 5, dans lequel le progestatif est de la trimégestone.

7. Procédé suivant la revendication 6, dans lequel la dose quotidienne de trimégestone est de 250 µg.

8. Procédé suivant la revendication 6, dans lequel la dose quotidienne de trimégestone est de 125 µg.

9. Procédé suivant la revendication 6, dans lequel la dose quotidienne de trimégestone est de 75 µg.

10. Procédé suivant la revendication 6, dans lequel la dose quotidienne de trimégestone est de 50 µg.

11. Procédé suivant la revendication 6, dans lequel la dose quotidienne de trimégestone est de 40 µg.

12. Procédé suivant la revendication 5, dans lequel le progestatif est du diénogest.

13. Procédé suivant la revendication 12, dans lequel la dose quotidienne de diénogest est de 2 mg.

14. Procédé suivant la revendication 12, dans lequel la dose quotidienne de diénogest est de 1 mg.

15. Procédé suivant la revendication 12, dans lequel la dose quotidienne de diénogest est de 800 µg.

16. Procédé suivant la revendication 12, dans lequel la dose quotidienne de diénogest est de 750 µg.

17. Procédé suivant la revendication 12, dans lequel la dose quotidienne de diénogest est de 500 µg.

18. Procédé suivant la revendication 1, dans lequel un placebo non contraceptif est administré pendant 3-5 jours consécutifs en commençant le jour immédiatement après le dernier jour d'administration de la combinaison de progestatif/oestrogène.

19. Procédé suivant la revendication 18, dans lequel le placebo non contraceptif contient un complément de fer.

20. Kit de contraception, adapté pour une administration orale lors d'un cycle menstruel de 28 jours commençant le jour 1 du cycle menstruel, contenant des unités posologiques qui comprennent 23-25 unités posologiques distinctes, lesdites unités posologiques contenant chacune une combinaison d'un progestatif à une dose sélectionnée parmi 40-500 µg de trimégestone et 250 µg-4 mg de diénogest, et un oestrogène à une dose équivalente au niveau de l'activité d'oestrogènes à 10-20 µg d'éthinyloestradiol, dans lequel chacune des unités posologiques contient la même dose de progestatif et d'oestrogène, et facultativement 3-5 unités posologiques contenant chacune un placebo non contraceptif.

21. Kit de contraception suivant la revendication 20, dans lequel l'oestrogène est sélectionné parmi de l'éthinyloestradiol, du 17ß-oestradiol; des oestrogènes conjugués, USP; de l'oestrone ou un sel de celle-ci; et du mestranol.

22. Kit de contraception suivant la revendication 21, dans lequel l'oestrogène est de l'éthinyloestradiol.

23. Kit de contraception suivant la revendication 20, dans lequel le nombre d'unités posologiques est de 24.

24. Kit de contraception adapté pour une administration orale suivant la revendication 20, contenant des unités posologiques comprenant 28 unités posologiques distinctes, 23-25 desdites unités posologiques contenant chacune une combinaison d'un progestatif à une dose sélectionnée parmi 40-500 µg de trimégestone et 250 µg-4 mg de diénogest, et d'un oestrogène à un dose équivalente au niveau de l'activité d'oestrogènes à 10-20 µg d'éthinyloestradiol, dans lequel chacune des 23-25 unités posologiques contient la même dose de progestatif et d'oestrogène, et 3-5 desdites unités posologiques contenant chacune un placebo non contraceptif.
